(19) Europäisches Patentamt
European Patent Office
Office européen des brevets

(11) **EP 0 693 052 B1**

(12) **FASCICULE DE BREVET EUROPEEN**

(45) Date de publication et mention
de la délivrance du brevet:
**09.01.2002 Bulletin 2002/02**

(51) Int Cl.7: **C07C 209/62**, C07K 1/06,
C07K 1/12, B01J 23/44

(21) Numéro de dépôt: **94913152.8**

(22) Date de dépôt: **08.04.1994**

(86) Numéro de dépôt international:
**PCT/FR94/00397**

(87) Numéro de publication internationale:
**WO 94/24088 (27.10.1994 Gazette 1994/24)**

(54) **REACTIF DE DESALLYLATION, PROCEDE DE DESALLYLATION UTILISANT CE REACTIF**

DESALLYHERUNGSREAGENZ UND VERFAHREN ZUR DESALLYHERUNG UNTER
VERWENDUNG DIESES REAGENZES

DEALLYLATION REAGENT AND DEALLYLATION METHOD USING SAID REAGENT

(84) Etats contractants désignés:
**AT BE CH DE DK ES FR GB GR IE IT LI LU MC NL
PT SE**

(30) Priorité: **09.04.1993 FR 9304233**

(43) Date de publication de la demande:
**24.01.1996 Bulletin 1996/04**

(73) Titulaire: **RHODIA CHIMIE
92408 Courbevoie Cédex (FR)**

(72) Inventeurs:
• **BERNARD, Jean-Marie Route du Large
F-69440 Mornant (FR)**
• **BLART, Errol
F-94700 Maison-Alfort (FR)**
• **GENET, Jean-Pierre
F-91370 Verrières-le-Buisson (FR)**
• **LEMAIRE-AUDOIRE, Sandrine
F-92200 Neuilly-sur-Seine (FR)**
• **SAVIGNAC, Monique
F-911190 Gify/Yvette (FR)**

(74) Mandataire: **Ricalens, François et al
Rhodia Services, Direction de la Propriété
Industrielle, 40, rue de la Haie-Coq
93306 Aubervilliers Cedex (FR)**

(56) Documents cités:
**EP-A- 0 194 554        EP-A- 0 407 256
EP-A- 0 518 295        EP-A- 0 566 459
WO-A-92/19643**

• **CHEMICAL ABSTRACTS, vol. 109, no. 15, 10
Octobre 1988, Columbus, Ohio, US; abstract no.
129602w, R NOYORI ET AL. 'preparation of
oligonucleotides by
platinum-group-compound-mediated
deprotection of O-allyl and N-allyloxycarbonyl
protected nucleotides' page 753 ; & JP,A,6 270
392 (NIPPON ZEON CO. LTD.) 31 Mars 1987**
• **CANADIAN JOURNAL OF CHEMISTRY vol. 63,
no. 4 , Avril 1985 , OTTAWA CA pages 823 - 827
M KAMBER ET G JUST
'Gamma-phosphono-gamma-lactones. The use
of allyl esters as easily removable phosphonate
protecting groups'**

**Description**

La présente invention a pour objet un réactif et un procédé utile pour cliver une fonction protégée, à l'occasion d'une synthèse organique par un groupe allyle, d'avec celui-là.

Il est courant de protéger une molécule en bloquant les fonctions qui dans les conditions opératoires envisagées seraient réactives ou réputées telles par des groupes qualifiés de protecteur.

Ces techniques sont particulièrement utiles lors des synthèses peptidiques ou sur les (poly)nucléotides et les fonctions les plus couramment protégées sont les fonctions acides, alcool, amines ou thiol.

Ces techniques de protection sont surtout utilisées pour la synthèse des molécules complexes telles que les peptides d'au moins 2 acides aminés plus souvent d'au moins trois ,ou lorsque l'on utilise des acides aminés complexes c'est à dire ceux qui présente une fonction à hydrogène mobile (aminé, alcool y compris phénol, cycle azoté, thiol, .....) en plus d'une fonction acide et en plus d'une fonction amine. Elle sont aussi utiles pour les nucléotides, nucléosides, polynucléotides et les polynucléosides. Un groupe protecteur peut être également utile pour des molécules présentant au moins six atomes de carbone avantageusement douze, ou pour des molécules qui possèdent plusieurs fonctions au moins deux, avantageusement trois fonctions.

Parmi les groupes les plus couramment utilisés, on peut citer le groupe BOC ou terbutyloxycarbonyle, les groupes Z ou benzyloxyle, voire même le groupe FMOC. Il convient de signaler que les groupes de structure allylique auraient été considérés comme potentiellement très intéressants si l'on disposait de moyen de clivage adapté.

A ce jour les seuls groupes protecteurs de type allylique qui soient utilisés sont les groupes allyloxycarbonyles dont la libération a été traitée dans des demandes précédentes (notamment la demande déposée en France sous le N°92/04621 ainsi que la demande européenne correspondante EP-A-566459, publiée le 20 Octobre 1993) de la demanderesse et qui ne sont pas à confondre avec les dérivés allylés selon la présente demande; ces dérivés allyloxycarbonyle présentent un fonction carbonyle dérivant d'une fonction acide qui rend moins difficile le clivage.

La déprotection usuellement utilisée est une lyse en milieu acide, en général en milieu halohydrique anhydre (c'est-à-dire avec une teneur en eau en général inférieure à 1%, avantageusement à $10^{-3}$, de préférence à $10^{-4}$).

Cette technique présente toutefois de nombreux inconvénients. La réaction de clivage est parfois lente ou nécessite de gros excès de réactif. Les groupements alcoxyles ont tendance à se transformer en carbocation avec évolution vers les doubles liaisons lorsque cela est possible, ou vers des réactions d'alcoylation sur le noyau, ce qui est particulièrement gênant dans le cas des synthèses de peptides dont la séquence présente des résidus nucléophiles tels que des noyaux aromatiques (tryptophane, tyrosine, ....) ou soufrés (méthionine).

Cette technique n'est pas applicable dans le cas des groupes allyles et c'est la raison pour laquelle ces groupes qui par ailleurs présenteraient de nombreux avantages, ne sont pas utilisés.

La demande de brevet européen 407256 vise l'utilisation de phosphines rendues solubles par l'adjonction de fonctions sulfoniques. Cette demande montre la nécessité d'utiliser comme solvant des nitriles pour réaliser une réaction d'allylation à partir d'une grande variété de composés allylés, plus particulièrement les carbonates d'allyles, les carboxylates d'allyles sont exemplifiés. Les nucléophiles à allyler sont définis très largement mais les seuls qui fussent testés sont les acétylacétate (composé de type malonique), la morpholine (amines cycliques) et les azidures. Le document est silencieux sur la question de savoir ce qu'il advient des composés allylants par ailleurs les nucléophiles correspondant aux atomes de rang au moins égal à celui du phosphore n'ont jamais été testés. La question de la libération des atomes de la colonne de l'azote n'est pas envisagée.

La demande de brevet européen 518295 vise la protection en synthèse peptidique au moyen de groupe allyle mais ne décrit que la libération des allyles connectés à un carbonyloxyle. Par ailleurs, le seul nucléophile cité est la morpholine.

La demande PCT N° WO92/19643 vise également l'utilisation des allyles comme agents de protection mais ne décrit que la libération des allyles connectés à un carbonyloxyle. Cette libération est pour l'essentiel menée en utilisant l'hydrure d'alcoylétain. Une seule libération utilisant un nucléophile est cité, il s'agit d'une libération utilisant la pipéridine ou la morpholine.

La demande européenne publiée sous le n° 194554 vise la libération des oximes à partir de leurs dérivés O-allylés. Dans cette demande on utilise des acides carboxyliques dont le mode d'action est explicable par la nucléophilie pour certains d'entre eux. Seuls les résultats obtenus à partir de l'acide formique, pour lequel le mécanisme d'action ne s'explique probablement pas par la nucléophilie, mais plutôt par son caractère réducteur, démontrent une libération significative et relativement rapide.

Le Chemical Abstracts volume 109, n° 15, 10 octobre 1988, n° 129602w vise la libération des dérivés du type allyloxyle au moyen de métaux de la mine du platine.

L'article de M KAMBER et G. JUST publié dans le "Canadian Journal of Chemistry", volume 63, n° 4, Avril 1985, pages 823 - 827, ne vise que la libération d'esters allylique.

Aucun de ces documents ne vise des réactifs et des procédés susceptibles de libérer un atome de la colonne de l'azote directement lié à une fonction allylique.

C'est pourquoi, un des buts de la présente invention est de foumir un procédé et un réactif qui permettent le clivage d'un groupe protecteur allytique d'avec la fonction qu'il protège.

Un autre but de la présente invention est de fournir un procédé et un réactif qui évitent les réactions d'alcoylation des noyaux aromatiques.

Un autre but de la présente invention est de fournir un procédé et un réactif qui évitent les réactions d'alcoylation de fonction(s) appartenant à la même molécule et qualifiée(s) de nucléophile.

Ces buts et d'autres, qui apparaltront par la suite, sont atteints au moyen d'un réactif utile pour cliver les groupes, dits protecteurs, allyliques des fonctions qu'ils sont censés protéger

Le réactif selon la présente invention comporte:

a) un système de solvant(s);

b) un catalyseur comportant au moins un élément de la colonne VIII de la classification périodique des éléments, ledit élément de la colonne VIII de la classification périodique étant coordonné par au moins un agent de coordination soluble dans le dit système de solvant(s);

c) un composé soluble au moins partiellement dans ledit système de solvant(s) et portant au moins une fonction nucléophile,

caractérisé par le fait que ledit nucléophile présente une fonction au moins aussi nucléophile que la diéthylamine et ladite fonction présentant elle-même comme atome nuciéophile, des atomes d'une ligne de la classification périodique au moins égale à la troisième et par le fait que lesdits agents de coordination sont des dérivés trivalents hydrocarbonés des éléments de la colonne VB choisis parmi :

soit des pnictines basiques dérivant des triarylphosphines par remplacements d'aryle par des chaînes dont les éventuelles insaturations sont non conjuguées avec les doublets de l'élément de la colonne V;

soit des pnictines polyfonctionnelles, en général bifonctionelle, permettant une chélation du métal par les fonctions pnictines.

Le système de solvant peut être monophasé ou biphasé(c'est à dire avec une phase liquide aqueuse et une phase organique).

Dans le cas d'un système biphasé, lorsque le substrat et/ou le produit d'arrivée sont peu solubles en phase aqueuse, il est possible de mener la réaction, ou bien en ajoutant un tiers solvant B, ou bien en un système multiphasé, soit sans adjonction, soit en ajoutant un solvant A, soit en ajoutant simultanément un tiers solvant B et solvant A.

Les solvants A sont des solvants organiques choisis de manière qu'ils dissolvent au moins 1 %, avantageusement au moins 2 %, de préférence 5% en masse du substrat et sont suffisamment hydrophobes pour n'être pas miscibles à l'eau en toute proportion.

Il est préférable que l'eau ne puisse dissoudre qu'au plus 10 % de solvant A, avantageusement au plus 1 %, en masse et ce même en présence du substrat en tant que tiers solvant.

Il est préférable que le solvant A ne puisse dissoudre qu'au plus 10% d'eau, avantageusement au plus 1 %, en masse et ce même en présence du substrat en tant que tiers solvant.

Les solvants A peuvent être des mélanges, y compris des fractions pétrolières. Naturellement, dans les conditions opératoires les solvants A doivent être inertes vis-à-vis des substrats et des réactifs utilisés.

Notamment lorsque le système de solvant est un système biphasique, on peut ajouter des agents de transfert de phase qui se répartiront entre les deux phases.

Les familles préférées de solvants sont choisies dans le groupe constitué par les hydrocarbures, les dérivés aromatiques, les éthers, les esters et les solvants halogénés. Si l'on désire récupérer ces solvants il est souhaitable qu'ils soient moins nucléophiles que ledit composé nucléophile, de manière à ne pas interférer avec la réaction à moins, bien sur que ledit composé nucléophile soit en excès suffisant pour jouer un rôle de (tiers)solvant.

A titre de paradigmes de ces familles, on peut citer comme dérivés aliphatiques halogénés le dichlorométhane, le dichloro-1,2-éthane, le trichloro-1,1,1- éthane, comme dérivés aromatiques le toluène et comme dérivés aromatiques halogénés le chlorobenzène, comme esters l'acétate d'éthyle et l'acétate d'isopropyle, comme éthers, l'oxyde de tertiobutyle et de méthyle ainsi que l'anisole et les alcools lourds, c'est-à-dire répondant aux contraintes d'immiscibilité comme spécifié ci-dessus.

Pour des raisons d'économie industrielle, il est préférable que le solvant A soit distillable sous pression atmosphérique ou sous vide primaire ou secondaire.

Parmi les solvants A il convient de citer particulièrement ceux qui sont phénoliques et qui sont détaillés dans les demandes Françaises de la demanderesse déposées sous les N°89/15957 et 91/12524 ainsi que les demandes européenes correspondantes EP-A-432022 et EP-A-537089.

Selon un mode de réalisation de la présente invention, lorsque les substrats ne sont pas hydrosolubles, il est alors loisible à l'homme de métier de rajouter un tiers solvant B dont le rôle sera de solubiliser le substrat dans la phase aqueuse.

Ce tiers solvant B pourra se partager entre phases aqueuse et. organique lorsque cette dernière existe, soit initialement, soit du fait de l'éventuel emploi simultané du solvant A.

Il est préférable que l'eau puisse dissoudre au moins 1/10 de tiers solvant B, avantageusement au moins 1/3, en masse et ce même en présence du catalyseur avec ses agents de coordination.

Avantageusement, on ajoute le tiers solvant en quantité suffisante pour que la quantité de substrat soluble dans la phase aqueuse soit au moins du même ordre de grandeur que la quantité de catalyseur présent dans la phase aqueuse en début de réaction.

Lorsque l'on utilise des systèmes de solvant(s) monophasés on peut utiliser les solvants qui sont utilisés comme tiers solvant dans le cas du système biphasé.

Ainsi parmi les solvants utilisables comme solvant ou tiers solvant, on peut citer les solvants hydrosolubles de types alcool, nitrile, éther (surtout cyclique), acide, sulfone, sulfoxyde, amide simple ou polyfonctionnel (comme l'urée), ester, cétone, voire aminé notamment dans le cas où ledit composé nucléophile sert aussi de (tiers)solvant.

Ainsi, on peut utiliser un système de solvant(s), monophasique ou non, comportant une phase hydrophile (c'est-à-dire comportant comme constituant principal un solvant, ou un mélange de solvants, miscible en toute proportion et elle-même miscible en de larges proportions).

Les métaux donnant les meilleurs résultats catalytiques sont les métaux de la mine du platine, de préférence ceux qui sont, ou bien isoélectroniques du palladium, ou bien à un état de valence qui soit isoélectronique du palladium zéro, de préférence les deux ; toutefois, il peut être économiquement intéressant d'utiliser des métaux moins lourds en raison de leur coût bien moindre ; au sein de la famille des métaux de la mine du platine, chacun d'entre eux présente des spécificités qui les rendent plus ou moins intéressants selon les cas ; le palladium, notamment à l'état d'oxydation zéro donne le plus souvent les meilleurs résultats.

Lesdits agents de coordination sont des dérivés trivalents hydrocarbonés des éléments de la colonne VB, avantageusement de période d'un rang supérieur à la deuxième et en général inférieur à la sixième, de la classification périodique des éléments (supplément au Bulletin de la Société Chimique de France Janvier 1966 N°1). Outre ceux qui sont détaillés par la suite on peut donner comme exemples de tels composés les dérivés des acides oxygénés trivalents (phosphoreux, arsénieux, antimonieux et pour mémoire nitreux) dérivés obtenus notamment par estérification ou par substitution d'au plus deux des trois oxhydryles (la trisubstitution conduit en fait aux pnictines qui fait l'objet d'une description plus détaillée).

Parmi les dits dérivés hydrocarbonés des éléments de la colonne V les préférés sont ceux qui dérivent des pnictures d'hydrogène par substitution totale ou partielle de l'hydrogène par des restes hydrocarbonés qui peuvent être reliés à l'atome de la colonne VB par une double (comme dans les imines) ou une triple liaison (comme dans les nitriles).

Toutefois les dérivés hydrocarbonés des éléments de la colonne V dérivent avantageusement des pnictures d'hydrogène par substitution totale, ou partielle, de l'hydrogène par des restes hydrocarbonés monovalents avantageusement par des alcoyles [dans la présente description ALCO-*yle* est pris dans son sans étymologique de reste hydrocarboné d'un ALCO-*ol* après ignorance de la fonction alcool (ou ol)] ; ces composés alcoylés seront, par analogie avec le terme de pnicture, désignés dans la présente description, sous le terme de pnictines.

Ainsi dans le cas de l'azote la substitution du nitrure d'hydrogène (ammoniac) donne les amines, dans le cas du phosphore la substitution du phosphure d'hydrogène donne les phosphines, dans le cas de l'arsenic la substitution de l'arséniure d'hydrogène donne les arsines et dans le cas de l'antimoine la substitution de l'antimoniure (ou stibiure) d'hydrogène donne les stibines. Ils sont avantageusement choisis parmi les dérivés hydrocarbonés du phosphore tels que les phosphines.

Avantageusement ladit catalyseur comporte comme agent de caoidination hydrosoluble une pnictine, une trialcoylphosphine, de préférence (pour des raisons économiques) une triarylphosphine, en général une triphénylphosphine. Ladite phosphine et ledit métal de la colonne VIII sont avantageusement sous forme de tétrakis phosphine métal.

Lorsque le système de solvant(s) le nécessite, il est utile et parfois nécessaire de rendre le système catalytique hydrosoluble; pour rendre solubles les agents de coordination et notamment les pnictines, il convient de greffer des groupes hydrosolubilisants polaires.

On peut greffer des groupements neutres tels que les polyols, mais compte tenu de la forte lipophilicité des pnictines, il est préférable que les groupes greffés soient ioniques, cationiques comme les ammoniums quaternaires ou anioniques, comme tout groupe constituant la base associée des acides de préférence forts. Dans ce demier cas, on peut citer les groupes carboxyliques sulfoniques, phosphoniques, et plus généralement ceux donnant une hydrophilie équivalente.

On peut notamment citer les groupes utilisés pour modifier les phosphines pour obtenir celles visées dans le brevet français déposé sous le n° 76/22824 et publié sous le n° 2.366.237 ou dans le brevet français publié sous le n° 2.549.840.

A titre de paradigme des phosphines hydrosolubles, on peut citer les triphénylphosphinetrisulfonates P $(C_6H_4-SO_3^-)_3$ solubles, par exemple alcalins et celles de formule $P(C_6H_4-CO_2H)_3$, de préférence sous forme anionique.

Ainsi selon une mise en oeuvre particulièrement intéressante de la présente invention, on peut utiliser un système biphasique dans lequel une des 2 phases liquides est une phase aqueuse, dans laquelle le métal de la colonne VIII est solubilisé en phase aqueuse par une pnictine, ou équivalent, hydrosoluble.

Lorsqu'il y a des risques d'empoisonnernent du catalyseur, c'est à dire lorsque on utilise des nucléophiles dits "mou", en général dont la fonction nucléophile est à base de métalloïdes de rang élevé au moins égal à celui du phosphore ou du soufre, il est préférable d'utiliser :

■  soit des pnictines, en général des phasphines dont la basicité est étevée (la basicité qui est faible avec les triarylphosphines, croît avec le nombre de remplacements d'aryle par des chaînes dont les éventuelles insaturations [leur présence n'est pas souhaitable] sont non conjuguées avec les doublets de l'élément de la colonne V), comme par exemples les bis- ou tri cyclohexylphosphines;

■  soit des pnictines polyfonctionnelles, en général bifonctionelle, permettant une chélation du métal par les fonctions pnictines; en général les fonctions pnictines sont, en prenant le chemin le plus direct, séparées par 2, 3 ou 4 atomes, le plus souvent de carbone ; les formules du type $\omega$, $\omega'$diphenylphosphinoéthane ; ou $\omega$, $\omega'$diphenylphos-phinobutane.

La technique biphasique facilite grandement la récupération et le recyclage du catalyseur, lequel recyclage est un des paramètres clés de la rentabilité de ce type de procédé en raison du prix toujours accru des métaux de la mine du platine.

Dans le cadre de la présente invention, on peut utiliser un catalyseur métallique sous forme élémentaire (degré d'oxydation zéro) ou sous forme oxydée. Ces catalyseurs peuvent se présenter sous forme de sels, d'oxydes ou de complexes. Parmi les sels, oxydes et complexes des métaux précédemment cités, à l'état d'oxydation II, on peut mentionner les chlorures de palladium, l'acétate de palladium, le chlorure de palladium complexé par le benzonitrile. Il convient de souligner que les anions sont de faible importance, seuls les cations comptent.

Parmi les complexes des métaux à l'état d'oxydation zéro, on peut citer le palladium dibenzylidèneacétone.

Il convient de souligner que le niveau d'oxydation du métal n'est pas nécessairement conservé dans le réacteur; en effet les pnictines sont en général assez réductrices pour réduire le palladium à l'état élémentaire, même introduit sous forme palladeuse.

Pour une meilleure mise en oeuvre de l'invention, on préfère utiliser une quantité de catalyseur telle que le rapport molaire entre le catalyseur métallique et les composés des éléments de la colonne V, lorsque ces demiers composés sont sous forme d'agents de coordination, trop souvent désignés sous l'expression anglo-saxonne de Ligand, soit compris entre 2 et 100, plus généralement de 4 à 30. Ces rapports molaires doivent tenir compte du nombre de fonctions coordinantes par molécule ; ainsi lorsqu'on utilise comme agent de coordination des molécules ayant deux fonctions pnictine, il convient de diviser par deux les valeurs des domaines ci-dessus.

La quantité de système de solvant(s) utilisée est telle que la concentration du métal de la colonne VIII soit de préférence supérieure à $10^{-5}$, avantageusement de $10^{-2}$ à $10^{-3}$ M dans le solvant

Ledit composé nucléophile doit présenter deux caractéristiques; il doit d'une part être nucléophile, c'est-à-dire riche en électrons et d'autre part être soluble dans le système de solvant(s).

Dans la présente demande on utilise ceux qui sur diverses échelles de nucléophilies en ont une au moins égale à celle de la diéthylamine (voir le "March" $3^{ème}$ édition pp 307-309)

En fait le choix du nucléophile dépend des fonctions à déprotéger en général on préfère utiliser comme nucléophiles, des molécules porteuses de fonction(s) au moins aussi nucléophile(s) que les fonctions vis-à-vis desquelles il convient d'être sélectif.

Lorsque les réactifs s'y prêtent, selon une mise en oeuvre particulièrement intéressante de la présente invention, on protone les fonctions dont l'alcoylation est à éviter et on choisit un nucléophile non protonable dans les conditions opératoires. Dans cette hypothèse la contrainte ci-dessus sur le caractère nucléophile devient que la nucléophilie doit être supérieure à celle de l'ion $NH_4^+$. Cette protonation se fait en phase aqueuse au moyen d'acide dont le pKa est inférieur d'au moins 1 point, de préférence d'au moins 2 points au pKa de l'acide associé à la fonction nucléophile que l'on désire protéger. Un excès d'au moins 10% par rapport à la quantité nécessaire à la neutralisation est préférable

Ces fonctions nucléophiles sont de préférence celles dont au moins une formule mésomères porte un hydrogène; Elles peuvent être des anions ou de molécules neutres. Pour illustrer la richesse de cette catégorie de substrat, on peut citer de façon non limitative:

-  les fonctions porteuses de soufre divalent (telle que thiol, ou polysulfure, thioacides,.....), de préférence celles dont au moins une formule mésomères porte un hydrogène:

- les sulfures et disulfures organiques aliphatiques (à radicaux primaires, secondaires ou tertiaires), aromatiques ou hétérocycliques ;
- les thiols (libres ou sous forme de sel);

- les pnictines (phosphines, ...) de préférence secondaires.

Certaines fonctions comme les pnictines primaires peuvent servir plusieurs (le plus souvent deux comme dans le cas des exemple ci dessus) fois de nucléophiles et ce fait doit être pris en compte pour le calcul de la quantité stoechiométrique.

Les fonctions nucléophiles sont celles qui correspondent à des atomes d'une ligne de la classification périodique au moins égale à la troisième comme le soufre par exemple de la fonction hydrogénosulure.

Notamment lorsque la fonction à desallyler est une base de type pnictine il est souhaitable de réaliser la réaction en milieu acide de Lewis, ou de preférence de Bronstedt.

Lorsque le substrat ne possède pas de fonction acide adéquate, il est préférable d'utiliser un acide dont le pka démontre une acidité d'au moins une unité, de préférence au moins deux unités, supérieure à l'acidité de l'acide associé à ladite base, il est préférable qu'au moins 50 %, plus avantageusement 90 % de ladite base soit sous forme protonée. L'acide peut être apporté par la même molécule que le nucléophile, ainsi que cela est mentionné ci après.

Lorsque cela est désiré, pour être hydrophile, ou plutôt hydrosoluble, c'est à dire qu'il soit soluble dans l'eau, ledit composé nucléophile doit être tel que dans les conditions normales l'eau soit capable d'en dissoudre au moins 0,2 avantageusement 0,5 de préférence 1 équivalent de fonction nucléophile.

Il est à noter que l'on peut rendre hydrosolubles des réactifs nucléophiles qui ne le seraient pas ou peu eu prévoyant une fonction fortement hydrophile sur la molécule. Les fonctions acides forte ou moyenne (pKa au plus égal à 6, avantageusement à 5, de préférence à 4), qu'elles soient sulfurées, (sulfonique, monoester sulfurique,....), phosphorées (ester phosphorique, acide phosphonique, acide phosphinique,.....), carbonées, ou autres, donnent des résultats suffisamment bons pour que l'on se demande s'il n'y a pas de synergie. Les meilleurs résultats sont à ce jour obtenus avec une fonction carbonée à savoir la fonction carboxylique.

Ainsi, avantageusement, les résultats obtenus à partir de nucléophiles où la fonction nucléophile est portée par un carbone postvicinal ou de préférence vicinal de celui portant la fonction acide.

D'une manière générale, il est préférable que, entre l'atome portant l'hydrogène acide (ou l'un des atomes portant l'hydrogène acide s'il y a plusieurs fonctions acides) et l'atome nucléophile, et y compris ces deux atomes, il ait, par le chemin le plus court, un nombre de chaînons compris entre 3 et 7, de préférence entre 4 et 6.

L'un des meilleurs nucléophiles est donc l'acide thiosalicylique (H-S Φ-COOH)(nombre de chaînons égal à 5), notamment sous forme monoanionique ou, de préférence acide.

Les meilleurs résultats sont ceux qui sont obtenus par l'association des fonctions nucléophiles non ou difficilement protonables avec les fonctions acides. Les résultats obtenus avec les zwitterions sont parfois médiocres.

Il est, bien sûr, préférable que l'eau et le nucléophile soient miscibles en toute proportion. Un même composé peut porter plusieurs fonctions nucléophiles

Il est préférable qu'il y ait, rapporté à au nombre de carbone au moins une fonction nucléophile pour 10 atomes de carbone avantageusement une pour 8 de préférence une pour 4.

Il est également préférable d'utiliser de petites molécules dont le nombre de carbone ne soit pas significativement supérieur à la dizaine.

Enfin il peut être pratique de choisir un composé nucléophile qui voit sa solubilité décroître considérablement avec la température pour passer en phase gazeuse permettant ainsi un déplacement aisé par distillation.

En général ledit composé nucléophile est présent (initialement mais plus préférablement en fin de réaction) à une concentration d'au moins 1/2, avantageusement 2, de préférence 5 équivalent par litre.

Lorsque le composé nucléophile présente, ramenés à une fonction nucléophile une masse moléculaire faible, des concentrations aussi élevées que 10 équivalents par gramme sont souvent dépassées.

Lorsque la desallylation apportée par l'utilisation de la phase aqueuse n'est pas jugée suffisante et que l'on désire l'augmenter, on peut pour ce faire jouer sur l'excès dudit composé nucléophile par rapport au substrat, par exemple en augmentant l'excès stoechiométrique (en général largement supérieur à 10%) par rapport à la réaction désirée pour le porter à une valeur au moins égale à 1/4, avantageusement à 1/2 de préférence à un fois de la quantité stoechiométrique (c'est à dire travailler avec des quantités respectivement au moins égales à 5/4 ; 3/2 et 2 Q.S.).

Ainsi, avantageusement selon la présente invention, la quantité du dit nucléophile est au moins égale à 3/2 fois la quantité stoechiométriquement nécessaire. En général de tels excès stoechiométrique ne sont utilisés que lorsque l'on désira un desallylation totale.

Un autre but de la présente invention est de foumir un procédé qui accélère sensiblement la cinétique de clivage.

Un autre but de la présente invention est de fournir un procédé qui évite les réactions d'akoylation des noyaux aromatiques.

Un autre but de la présente invention est de fournir un procédé qui évite les réactions d'alcoylation des fonctions qualifiées de nucléophile.

Ces buts et d'autres, qui apparaitront par la suite, sont atteints au moyen d'un procédé de traitement de molécule comportant au moins une fonction allyle reliée directement à un atome de la colonne V, où ladite molécule est soumise à l'action d'un milieu réactionnel qui comporte

a) un système de solvant(s) ;

b) un catalyseur comportant au moins un élément de la colonne VIII de la classification périodique des éléments, ledit élément de la colonne VIII de la classification périodique étant coordonné par au moins un agent de coordination soluble dans le dit système de solvant(s);

c) un composé soluble au moins partiellement dans ledit système de solvant(s) et portant au moins une fonction au moins aussi nucléophile que la diéthylamine, ladite fonction présentant elle-même comme atome nucléophile, des atomes d'une ligne de la classification périodique au moins égale à la troisième.

Avantageusement lesdites molécules comportent au moins une fonction allyle répondant à la formule suivante (I) :

$$Z-C(R_1)(R_2)-C(R_3)=C(R_4)(R_5) \qquad (I)$$

où

$R_1$ représente un hydrogène ou un radical alcoyle, de préférence à 1 ou 2 atomes de carbone;

$R_2$ représente un hydrogène ou un radical alcoyle, de préférence à 1 ou 2 atomes de carbone ;

$R_3$ représente un hydrogène ou un radical alcoyle, de préférence à 1 ou 2 atomes de carbone;

$R_4$ représente un hydrogène ou un radical alcoyle, de préférence à 1 ou 2 atomes de carbone;

$R_5$ représente un hydrogène ou un radical alcoyle, de préférence à 1 ou 2 atomes de carbone ou aryle;

Z étant le radical issu de la molécule à protéger, la liaison remplaçant un hydrogène de la fonction dont on désire la protection.

Z contient la molécule protégée et qu'il faut libérer de son groupe protecteur.

Ladite fonction à protéger n'étant pas une équivalente d'une fonction acide.

$R_5$ peut être un groupe dit "Ar" tel que décrit dans la demande de brevet britannique déposée le 31.12.1990 sous le N°90 28208.8 publiée sous le N° 2251242 et intitulée "groupe Protecteur".

$R_5$ et $R_4$ peuvent être des fractions du groupe dit "Ar" dans la demande ci-dessus de manière que $R_5$ et $R_4$ ainsi que le carbone qui les porte forme un radical Ar tel que défini dans la demande britannique ci dessus.

$R_5$ peut être tout groupe lipophile tel que décrit dans les demandes de brevets français au nom de la demanderesse déposées le 02.10.1989 sous le numéro N°89/13054 et intitulée "Procédé de solubilisation de peptides et procédé de synthèse de peptides." et celle déposée le 04.12.1989 sous le N°89/15957 et intitulée "Milieu réactionnel et solubilisant de peptides et procédé de synthèse utilisant ce milieu." ainsi que les demandes européenes correspondantes EP-A-421848 et EP-A-432022.

D'une manière générale l'association du groupe désigné par Ar dans la demande britannique ci-dessus avec un groupement allyle en tant que "L" est très favorable.

Il est préférable que Z soit de structure Z'-χ- avec χ étant un atome de la colonne V, de préférence un azote (lui même avantageusement alcoylé de manière que dans la formule finale l'azote soit tertiaire, si l'on désire faciliter le clivage). Z' n'a avantageusement pas de fonction carbonyle liée à χ.

Le terme "alcoylé" est pris dans son sens étymologique déjà spécifié avec la précision supplémentaire qu'il peut également signifier un groupement aryle.

Il est préférable qu'au moins 2, avantageusement 3, de préférence 4 des radicaux R1 à R5 soient au plus de deux carbones ; toutefois, au moins un des radicaux R1 à R5 peut être tel que l'alcool allylique soit un alcool lourd, par exemple de série aromatique, de type terpénique ou de série stéroïdienne.

Ainsi, au moins 1 radical et au plus 3 radicaux R1 à R5 peuvent être des radicaux aryles polycycliques, condensés ou non, homo ou hétérocycliques.

Ainsi le présente invention permet d'utiliser des molécules de structure :

$$\zeta(\text{-Allyl}_i)_n$$

avec i prenant toute les valeurs entières de 1 à n

- allyl$_i$ étant de formule

$$-C(R_1)(R_2)-C(R_3)=C(R_4)(R_5)$$

Les allyl$_i$ étant tels qu'il y ait dans la molécule avantageusement au moins deux, de préférence 3, formules différentes de allyl$_i$. Il est préférable que ces formules présentent respectivement 0,1 et /ou 2 substituants $R_1$ à $R_5$.

n est avantageusement au moins égal à deux, de préférence à 3 et n'est en général au plus égal à 10 (un chiffre significatif).

La température réactionnelle est en général comprise entre le point de fusion finissante et le point d'ébullition commençante du milieu réactionnel. Avantageusement entre 0°C et 100°C de préférence entre l'ambiante (environ 20°C) et 50°C.

Il apparaît que la sélectivité croît lorsque la réaction est menée dans deux phases mais la cinétique décroît.

Selon la présente invention il a été trouvé que l'on pouvait utiliser les dérivés allylés directs (directs par opposition aux dérivés allyloxycarbonyles où par exemple la fonction amine est protégée sous la forme d'un allyloxycarbamate, la rupture de la fonction ester induisant celle de la fonction carbamique, libérant ipso facto la fonction amine), c'est à dire ne possédant pas de fonction carbonyle en position post homoallylique facilitant le clivage (comme par exemple dans le cas des carboxylates d'allyle), pour protéger diverses fonctions possédant un hydrogène mobile. Il a été notamment prouvé que l'on pouvait protéger une fonction amine primaire au moyen de deux fonctions allyles.

Il a été en outre démontré que les diverses fonctions allyles se desallylaient sélectivement les unes par rapport aux autres. Cette sélectivité ne joue pas uniquement entre allyles diversement substitués, mais aussi entre groupes allyles identiques, par exemple entre allyles sensu stricto ou entre methallyles. Cette propriété permet de réaliser une désallylation des amines tertiaires (notamment celles au moins diallylées) qui soit sélective par rapport à celle des amines secondaires (notamment celles au moins mono allylées). Cela permet notamment de réaliser une mono desallylation sur un atome de la colonne V, notamment un azote, lorsque celui-ci porte aux moins deux fonctions allyliques.

Ainsi en jouant sur la nucléophilie des composés récepteurs des groupes allyle, ou en jouant sur la quantité du dit récepteur, il est possible de libérer successivement les fonctions allylées suivantes :

- Pour mémoire les fonctions allyloxycarbonyles (pour celle-ci des foncions amines donne de bon résultats comme nucléophile)
- les fonctions allylées tertiaires ;
- les fonctions allylées secondaire.

Ainsi lorsque l'on désire une desallylation qui soit sélective on peut soit:

- de préférence n'utiliser que la seule quantité stoechiométrique (plus ou moins 10%) nécessaire à la première desallylation ;
- choisir un récepteur peu nucléophile;
- choisir des pnictines peu complexantes;
- mener la réaction à faible température ;
- Soit enfin combiner tout ou partie des mesures ci-dessus.

En général on préfère choisir la première mesure.

Cet sélectivité surprenante permet réaliser des synthèses jusque là difficiles. Ainsi la synthèse d'une aminé trisubstituée différemment, peut être réalisée en partant d'une amine diallylée (qui peut être obtenue soit par action d'agent allylant sur une amine primaire soit d'agent alcoylant sur une diallylamine, en général la diallyamine stricto sensu)

- en procédant à une monodésallylation sélective selon la présente invention ;
- en greffant un nouveau radical sur l'amine ;
- en libérant la deuxième fonction allyle ;
- éventuellement en graffant un autre radical sur l'amine secondaire ainsi formée.

Le procédé selon l'invention respecte la géométrie des molecules et est de ce fait particulièrement bien adapté à la chimie des molécules chirales.

Les exemples non limitatifs suivants illustrent l'invention.

Définitions

TT = Taux de Transformation :

$$TT = \frac{\text{nombre de moles de produit transformé}}{\text{nombre de moles de produit initial}}$$

RR = le rendement sur le produit initial

$$RR = \frac{\text{nombre de moles de produit final}}{\text{nombre de moles de produit initial}}$$

RT = le rendement sur le produit transformé

$$RT = \frac{\text{nombre de moles de produit final}}{\text{nombre de moles de produit transformé.}}$$

DEPROTECTION EN MILIEU MONOPHASE FAIBLEMENT AQUEUX

Mode opératoire général:

Le catalyseur est préformé en agitant 15 minutes, sous argon, le Pd(dba)$_2$ et le coordinant diphénylphosphi-nobutane (D.P.P.B.) (1:1) dans 1 ml de THF anhydre (2,5 à 5 % de catalyseur pour la réaction). On obtient alors une solution brun-roux.

Dans un tube, sous argon, sont introduits le substrat à déprotéger (100 à 500 mg) et 3 ml de THF anhydre. Puis sont additionnés le nucléophile (composé soufré) ou la diéthylamine (dégazée), et enfin le système catalytique préformé.

Lorsque le nucléophile est la diéthylamine, le milieu réactionnel est filtré sur gel de silice (éluant : AcOEt/ cyclohexane (1:1). Le filtrat est ensuite concentré sous pression réduite.

Lorsque le nucléophile est l'acide mercapto-2-benzoïque, ce dernier peut être séparé du produit déprotégé par traitement avec une solution basique (NaOH 10 %) au cours d'une extraction à l'acétate d'éthyle. La phase organique est séchée puis concentrée. Les produits déprotégés peuvent être purifiés par chromatographie sur gel de silice ou distillés.

Exemple 1

Le mode opératoire ci dessus a été suivi pour la méthyl allyl benzylamine en utilisant comme nucléophile l'acide mercapto-2-benzoïque.

Au bout de 15 minute à température ambiante 81% (RR = 81%; RT = 100 %; TT = 81 %) du substrat a été libéré.

Exemple 2

Ce mode opératoire de l'exemple 1 a été appliqué dans les conditions mentionnées dans les quatre tableaux ci-après, les résultats sont rassemblés dans ces mêmes tableaux.

Déprotection des allylamines dérivant d'amines secondaires

| Substrat | Produit | Temps | Rdt (%) |
|---|---|---|---|
| 1. | | 25 min | 100 |
| 2. | | 40 min | 97[(*)] |
| 3. | | 30 min | 100 |
| 4. | | 15 min | 100 |
| 5. | | 10 min | 93 |
| 6. | | 15 min | 100 |
| 7. | | 15 min | 100 |

(*) : 2 eq d'acide 2-mercapto-benzoïque sont utilisés

Tableau 1

<u>Désallylation totale des mono et des diallylamines</u>

(a) R' = H, acide 2-mercapto-benzoïque 1,1 éq.
(b) R' = allyl, acide 2-mercapto-benzoïque 2,1 éq.

| Substrat | Produit | Temps | Rdt (%) |
|---|---|---|---|
| 1. | | 30 min | 100 |
| 2. | | 15 min | 100 |
| 3. | | 20 min | 100 |
| 4. | | 15 min | 100 |
| 5. | | 30 min | 97 |
| 6. | | | |

Tableau 2

## Monodéprotection selective des diallylamines

| | Substrat | Produit | Temps | Rdt(%) |
|---|---|---|---|---|
| 1. | | | 30 min | 100 |
| 2. | | | 30 min | 100 |
| 3. | | | 30 min | 100 |
| 4. | | | 90 min | 98 |
| 5. | | | 30 min | 70 |
| 6. | | | 45 min | 93 |
| 7. | | | 30 min | 72 |
| 8. | | (82 : 18) | 15 min | |

Pd (0) = Pd(dba)$_2$ · DPPB

**Tableau 3**

## Déprotections chimiosélectives de molécules hydroxy-amines

Etape (1)
Pd (0), 5% mol

HNEt₂ 2 eq, THF

Etape (2)
Pd (0), 5% mol

SH
1,1 eq   THF
COOH

Etape (1)          Etape (2)

| Substrat | Produit | Temps/ Rdt(%) | Produit | Temps/ Rdt(%) |
|---|---|---|---|---|
| 1. | | 30 min/ 100 | | 30 min/ 100 |
| 2. | | 45 min/ 100 | | 30 min/ 100 |
| 3. | | 15 min/ 98 | | 15 min/ 100 |

Pd (0) = Pd(dba)₂ - DPPB

### Tableau 4

## Revendications

1. Réactif utile pour le clivage d'un groupe protecteur allylique d'avec la fonction qu'il protège comportant:

   a) un système de solvant(s);
   b) un catalyseur comportant au moins un élément de la colonne VIII de la classification périodique des éléments, ledit élément de la colonne VIII de la classification périodique étant coordonné par au moins un agent de coordination soluble dans ledit système de solvant(s);

c) un composé soluble au moins partiellement dans ledit système de solvant(s) et portant au moins une fonction nucléophile,

**caractérisé par le fait que** ledit nucléophile présente une fonction au moins aussi nucléophile que la diéthylamine et ladite fonction présentant elle-même comme atome nucléophile, des atomes d'une ligne de la classification périodique au moins égale à la troisième et **par le fait que** lesdits agents de coordination sont des dérivés trivalents hydrocarbonés des éléments de la colonne VB choisis parmi:

- □ soit des pnictines basiques dérivant des triarylphosphines par remplacements d'aryle par des chaînes dont les éventuelles insaturations sont non conjuguées avec les doublets de l'élément de la colonne V;
- □ soit des pnictines polyfonctionnelles, en général bifonctionelle, permettant une chélation du métal par les fonctions pnictines.

**2.** Réactif selon la revendication 1, **caractérisé par le fait que** lesdits agents de coordination sont des dérivés trivalents hydrocarbonés des éléments de la colonne VB de période d'un rang supérieur à la deuxième ligne.

**3.** Réactif selon les revendications 1 et 2, **caractérisé par le fait que** lesdits agents de coordination basique sont des trialcoylphosphines.

**4.** Réactif selon les revendications 1 à 3, **caractérisé par le fait que** les pnictines basiques sont des bis- ou tricyclohexylphosphines.

**5.** Réactif selon les revendications 1 à 4, **caractérisé par le fait que** lesdits agents de coordination sont des pnictines bifonctionelles, lesdites fonctions pnictines, avantageusement phosphines, étant, en prenant le chemin le plus direct, séparées par 2, 3 ou 4 atomes.

**6.** Réactif selon les revendications 1 à 5, **caractérisé par le fait que** lesdits agents de coordination sont choisis parmi l'ω, ω'diphenylphosphinoéthane et l'ω, ω'diphenylphosphinobutane.

**7.** Réactif selon les revendications 1 à 6, **caractérisé par le fait que** ledit nucléophile présente une fonction sulfure.

**8.** Réactif selon les revendications 1 à 7, **caractérisé par le fait que** ledit nucléophile présente une fonction acide dont le pKa est au plus égal à 6, avantageusement à 5, de préférence à 4.

**9.** Réactif selon la revendication 8, **caractérisé par le fait que** par le chemin le plus court, il a entre l'atome portant l'hydrogène acide (ou l'un des atomes portant l'hydrogène acide s'il y a plusieurs fonction acide) et l'atome nucléophile, et y compris ces deux atomes, un nombre de chaînons compris entre 3 et 7, de préférence entre 4 et 6.

**10.** Réactif selon les revendications 1 à 9, **caractérisé par le fait que** ledit nucléophile est un acide présentant une fonction sulfure, avantageusement l'acide thiosalicylique.

**11.** Procédé de traitement de molécule comportant au moins une fonction allyle reliée directement à un atome de la colonne V, où ladite molécule est soumise à l'action d'un milieu réactionnel qui comporte :

a) un système de solvant(s);

b) un catalyseur comportant au moins un élément de la colonne VIII de la classification périodique des éléments, ledit élément de la colonne VIII de la classification périodique étant coordonné par au moins un agent de coordination soluble dans le dit système de solvant(s);

c) un composé soluble au moins partiellement dans ledit système de solvant(s) et portant au moins une fonction au moins aussi nucléophile que la diéthylamine, ladite fonction présentant elle-même comme atome nucléophile, des atomes d'une ligne de la classification périodique au moins égale à la troisième.

**12.** Procédé selon la revendication 11, **caractérisé par le fait que** ladite molécule comporte au moins une fonction allyle répondant à la formule suivante (I):

$$Z\text{-}C(R_1)(R_2)\text{-}C(R_3)=C(R_4)(R_5) \tag{I}$$

Où:

- $R_1$ représente un hydrogène ou un radical alcoyle, de préférence à 1 ou 2 atomes de carbone ;
- $R_2$ représente un hydrogène ou un radical alcoyle, de préférence à 1 ou 2 atomes de carbone ;
- $R_3$ représente un hydrogène ou un radical alcoyle, de préférence à 1 ou 2 atomes de carbone ;
- $R_4$ représente un hydrogène ou un radical alcoyle, de préférence à 1 ou 2 atomes de carbone ;
- $R_5$ représente un hydrogène ou un radical alcoyle, de préférence à 1 ou 2 atomes de carbone ou aryle ;

Z étant le radical issu de la molécule à protéger la liaison remplaçant un hydrogène de la fonction dont on désire la protection. Z contient la molécule protégée et qu'il faut libérer de son groupe protecteur.

**13.** Procédé selon les revendications 11 et 12, **caractérisé par le fait que** ladite molécule présente au moins 2, et au plus 10 groupes allyles de formule:

$$-C(R_1)(R_2)\text{-}C(R_3)=C(R_4)(R_5).$$

**14.** Procédé selon la revendication 13, **caractérisé par le fait que** ledit procédé de traitement est une désallylation sélective.

**15.** Procédé selon les revendications 11 à 14, **caractérisé par le fait que** ledit procédé de traitement est partie d'une synthèse peptidique.

**16.** Procédé selon les revendications 11 à 15, **caractérisé par le fait que** ledit procédé de traitement est partie d'une synthèse de nucléotides ou de nucléosides.

**17.** Procédé selon les revendications 11 à 16, **caractérisé par le fait que** ledit procédé de traitement est une synthèse d'amine.

**18.** Procédé selon les revendications 11 à 17, **caractérisé par le fait que** la quantité dudit nucléophile est au moins égale à 3/2 fois la quantité stoechiométriquement nécessaire pour la désallylation.

**19.** Procédé selon les revendications 11 à 18, **caractérisé par le fait que** la quantité dudit nucléophile est au moins égale à une valeur comprise entre 0,9 et 1,1 fois la quantité stoechiométriquement nécessaire pour la désallylation.

**20.** Procédé selon les revendications 11 à 19, **caractérisé par le fait que** ledit nucléophile présente une fonction acide.

**21.** Procédé selon les revendications 11 à 20, **caractérisé par le fait que** ledit nucléophile présente une fonction nucléophile présentant elle-même un atome de soufre comme atome nucléophile.

**Patentansprüche**

**1.** Reagenz, das für die Abspaltung einer allylischen Schutzgruppe von der Funktion, die sie schützt, nützlich ist, das umfasst:

a) ein System von Lösemittel(n);
b) einen Katalysator, der mindestens ein Element der Gruppe VIII des Periodensystems der Elemente umfasst, wobei das Element der Gruppe VIII des Periodensystems mit mindestens einem Koordinationsmittel, das in dem System von Lösemittel(n) löslich ist, koordiniert ist;
c) eine Verbindung, die zumindest teilweise in dem System von Lösemittel(n) löslich ist und mindestens eine nukleophile Funktion trägt;

**dadurch gekennzeichnet, dass** das Nukleophil eine mindestens ebenso nukleophile Funktion wie Diethylamin aufweist, und wobei die Funktion ihrerseits als nukleophiles Atom Atome einer Zeile des Periodensystems, die mindestens gleich der dritten ist, aufweist, und dadurch, dass die Koordinationsmittel dreiwertige kohlenwasserstoffhaltige Derivate der Elemente der Gruppe VB sind, die ausgewählt sind unter:

- entweder basischen Pnictinen, die sich von Triarylphosphinen durch Ersetzungen von Aryl durch Ketten, deren gegebenenfalls vorhandene Ungesättigtheiten nicht mit den E-lektronendubletts des Elements der Gruppe V konjugiert sind, ableiten;
- oder polyfunktionellen, im allgemeinen bifunktionellen Pnictinen, die eine Komplexierung des Metalls durch die Pnictinfunktionen erlauben.

2. Reagenz nach Anspruch 1, **dadurch gekennzeichnet, dass** die Koordinationsmittel dreiwertige kohlenwasserstoffhaltige Derivate der Elemente der Gruppe VB einer Periode eines höheren Rangs als die zweite Zeile sind.

3. Reagenz nach den Ansprüchen 1 und 2, **dadurch gekennzeichnet, dass** die basischen Koordinationsmittel Trialkylphosphine sind.

4. Reagenz nach den Ansprüchen 1 bis 3, **dadurch gekennzeichnet, dass** die basischen Pnictine Bis- oder Tricyclohexylphosphine sind.

5. Reagenz nach den Ansprüchen 1 bis 4, **dadurch gekennzeichnet, dass** die Koordinationsmittel bifunktionelle Pnictine sind, wobei die Pnictinfunktionen, vorzugsweise Phosphinfunktionen, wenn man den direktesten Weg nimmt, durch 2, 3 oder 4 Atome voneinander getrennt sind.

6. Reagenz nach den Ansprüchen 1 bis 6, **dadurch gekennzeichnet, dass** die Koordinationsmittel unter $\omega$, $\omega'$-Diphenylphosphinoethan und $\omega,\omega'$-Diphenylphosphinobutan ausgewählt sind.

7. Reagenz nach den Ansprüchen 1 bis 6, **dadurch gekennzeichnet, dass** das Nukleophil eine Sulfidfunktion aufweist.

8. Reagenz nach den Ansprüchen 1 bis 7, **dadurch gekennzeichnet, dass** das Nukleophil eine Säurefunktion aufweist, deren pKa höchstens gleich 6, vorteilhafterweise höchstens gleich 5, vorzugsweise höchstens gleich 4 ist.

9. Reagenz nach Anspruch 8, **dadurch gekennzeichnet, dass** es auf dem kürzesten Weg zwischen dem Atom, das das saure Wasserstoffatom trägt, (oder zwischen einem der Atome, die das saure Wasserstoffatom tragen, wenn es mehrere Säurefunktionen gibt) und dem nukleophilen Atom, und einschließlich dieser zwei Atome, eine Anzahl von Kettengliedern zwischen 3 und 7, vorzugsweise zwischen 4 und 6 gibt.

10. Reagenz nach den Ansprüchen 1 bis 9, **dadurch gekennzeichnet, dass** das Nukleophil eine Säure, die eine Sulfidfunktion aufweist, vorteilhafterweise Thiosalicylsäure ist.

11. Verfahren zur Behandlung eines Moleküls, das mindestens eine allylische Funktion direkt an ein Atom der Gruppe V gebunden aufweist, worin das genannte Molekül der Wirkung eines Reaktionsmediums unterworfen wird, das umfasst:

a) ein System von Lösemittel(n);
b) einen Katalysator, der mindestens ein Element der Gruppe VIII des Periodensystems der Elemente umfasst, wobei das Element der Gruppe VIII des Periodensystems mit mindestens einem Koordinationsmittel, das in dem System von Lösemittel(n) löslich ist, koordiniert ist;
c) eine Verbindung, die zumindest teilweise in dem System von Lösemittel(n) löslich ist und mindestens eine Funktion trägt, die mindestens ebenso nukleophil wie Diethylamin ist, wobei diese Funktion ihrerseits als nukleophiles Atom Atome einer Zeile des Periodensystems, die mindestens gleich der Dritten ist, aufweist.

12. Verfahren nach Anspruch 11, **dadurch gekennzeichnet, dass** das Molekül mindestens eine allylische Funktion umfasst, die der folgenden Formel (I) entspricht:

$$Z-C(R_1)\,(R_2)-C(R_3)=C(R_4)\,(R_5) \qquad\qquad (I),$$

worin:

- $R_1$ für ein Wasserstoffatom oder einen Alkylrest, vorzugsweise mit 1 oder 2 Kohlenstoffatomen, steht;
- $R_2$ für ein Wasserstoffatom oder einen Alkylrest, vorzugsweise mit 1 oder 2 Kohlenstoffatomen, steht;
- $R_3$ für ein Wasserstoffatom oder einen Alkylrest, vorzugsweise mit 1 oder 2 Kohlenstoffatomen, steht;
- $R_4$ für ein Wasserstoffatom oder einen Alkylrest, vorzugsweise mit 1 oder 2 Kohlenstoffatomen, steht;
- $R_5$ für ein Wasserstoffatom oder einen Alkylrest, vorzugsweise mit 1 oder 2 Kohlenstoffatomen, oder einen Arylrest steht;

wobei Z der Rest ist, der aus dem zu schützenden Molekül stammt, wobei die Bindung ein Wasserstoffatom der Funktion, deren Schutz man wünscht, ersetzt. Z enthält das geschützte Molekül, das man von seiner Schutzgruppe befreien muss.

**13.** Verfahren nach den Ansprüchen 11 und 12, **dadurch gekennzeichnet, dass** das Molekül mindestens 2 und höchstens 10 allylische Gruppen der Formel:

$$-C(R_1)(R_2)-C(R_3)=C(R_4)(R_5)$$

aufweist.

**14.** Verfahren nach Anspruch 13, **dadurch gekennzeichnet, dass** das Behandlungsverfahren eine selektive Desallylierung ist.

**15.** Verfahren nach den Ansprüchen 11 bis 14, **dadurch gekennzeichnet, dass** das Behandlungsverfahren Teil einer Peptidsynthese ist.

**16.** Verfahren nach den Ansprüchen 11 bis 15, **dadurch gekennzeichnet, dass** das Behandlungsverfahren Teil einer Nukleotid- oder Nukleosidsynthese ist.

**17.** Verfahren nach den Ansprüchen 11 bis 16, **dadurch gekennzeichnet, dass** das Behandlungsverfahren eine Aminsynthese ist.

**18.** Verfahren nach den Ansprüchen 11 bis 17, **dadurch gekennzeichnet, dass** die Menge des Nukleophils mindestens gleich dem 3/2-fachen der für die Desallylierung benötigten stöchiometrischen Menge ist.

**19.** Verfahren nach den Ansprüchen 11 bis 18, **dadurch gekennzeichnet, dass** die Menge des Nukleophils mindestens gleich einem Wert zwischen dem 0,9- und 1,1-fachen der für die Desallylierung benötigten stöchiometrischen Menge ist.

**20.** Verfahren nach den Ansprüchen 11 bis 19, **dadurch gekennzeichnet, dass** das Nukleophil eine Säurefunktion aufweist.

**21.** Verfahren nach den Ansprüchen 11 bis 20, **dadurch gekennzeichnet, dass** das Nukleophil eine nukleophile Funktion, die ihrerseits als nukleophiles Atom ein Schwefelatom aufweist, aufweist.

**Claims**

**1.** Reagent which is useful for cleaving an allylic protecting group from the function it is protecting, comprising:

a) a solvent system;
b) a catalyst comprising at least one element from column VIII of the Periodic Table of the Elements, the said element from column VIII of the Periodic Table being coordinated with at least one coordination agent which is soluble in the said solvent system;
c) a compound which is at least partially soluble in the said solvent system and which bears at least one nucleophilic function,

**characterized in that** the said nucleophile has a function which is at least as nucleophilic as diethylamine and the said function itself having, as nucleophilic atom, atoms from a row of the Periodic Table at least equal to the third row and that the said coordination agents are trivalent hydrocarbon-based derivatives of elements from column VB, chosen from:

- either basic pnictines derived from triarylphosphines by replacements of aryl with chains in which the possible unsaturations are not conjugated with the lone pairs of the element from column V;
- or polyfunctional, generally difunctional, pnictines which allow chelation of the metal with the pnictine functions.

2. Reagent according to Claim 1, **characterized in that** the said coordination agents are trivalent hydrocarbon-based derivatives of elements from column VB which are from a period higher than the second row.

3. Reagent according to Claims 1 and 2, **characterized in that** the said basic coordination agents are trialkylphosphines.

4. Reagent according to Claims 1 to 3, **characterized in that** the basic pnictines are bis- or tricyclohexylphosphines.

5. Reagent according to Claims 1 to 4, **characterized in that** the said coordination agents are difunctional pnictines, the said pnictine functions, advantageously phosphines, being separated, taking the most direct path, by 2, 3 or 4 atoms.

6. Reagent according to Claims 1 to 5, **characterized in that** the coordination agents are chosen from $\omega,\omega'$-diphenylphosphinoethane and $\omega,\omega'$-diphenylphosphinobutane.

7. Reagent according to Claims 1 to 6, **characterized in that** the said nucleophile contains a sulphide function.

8. Reagent according to Claims 1 to 7, **characterized in that** the said nucleophile contains an acid function whose pKa is not more than 6, advantageously not more than 5 and preferably not more than 4.

9. Reagent according to Claim 8, **characterized in that**, via the shortest path, between the atom bearing the acidic hydrogen (or one of the atoms bearing the acidic hydrogen if there are several acid functions) and the nucleophilic atom, including these two atoms, there are between 3 and 7 and preferably between 4 and 6 chain units.

10. Reagent according to Claims 1 to 9, **characterized in that** the said nucleophile is an acid containing a sulphide function, advantageously thiosalicylic acid.

11. Process for treating a molecule comprising at least one allylic function linked directly to an atom from column V, in which the said molecule is subjected to the action of a reaction medium which comprises:

a) a solvent system;
b) a catalyst comprising at least one element from column VIII of the Periodic Table of the Elements, the said element from column VIII of the Periodic Table being coordinated with at least one coordination agent which is soluble in the said solvent system;
c) a compound which is at least partially soluble in the said solvent system and which bears at least one function which is as nucleophilic as diethylamine, the said function itself containing, as nucleophilic atom, atoms from a row of the Periodic Table at least equal to the third.

12. Process according to Claim 11, **characterized in that** the said molecule comprises at least one allylic function corresponding to formula (I) below:

$$Z\text{-}C(R_1)\,(R_2)\text{-}C(R_3)\text{=}C(R_4)\,(R_5) \qquad\qquad (I)$$

in which:

- $R_1$ represents a hydrogen or an alkyl radical, preferably containing 1 or 2 carbon atoms;
- $R_2$ represents a hydrogen or an alkyl radical, preferably containing 1 or 2 carbon atoms;

- $R_3$ represents a hydrogen or an alkyl radical, preferably containing 1 or 2 carbon atoms;
- $R_4$ represents a hydrogen or an alkyl radical, preferably containing 1 or 2 carbon atoms;
- $R_5$ represents a hydrogen or an alkyl radical, preferably containing 1 or 2 carbon atoms or an aryl radical;

Z being the radical derived from the molecule to be protected, the bond replacing a hydrogen of the function which it is desired to protect, Z contains the protected molecule which needs to be released from its protecting group.

13. Process according to either of Claims 11 and 12, **characterized in that** the said molecule contains at least 2 and not more than 10 allylic groups of formula:

$$-C(R_1)(R_2)-C(R_3)=C(R_4)(R_5).$$

14. Process according to Claim 13, **characterized in that** the said treatment process is a selective deallylation.

15. Process according to Claims 11 to 14, **characterized in that** the said treatment process is part of a peptide synthesis.

16. Process according to Claims 11 to 15, **characterized in that** the said treatment process is part of a nucleotide or nucleoside synthesis.

17. Process according to Claims 11 to 16, **characterized in that** the said treatment process is an amine synthesis.

18. Process according to Claims 11 to 17, **characterized in that** the amount of the said nucleophile is at least equal to 3/2 times the amount stoichiometrically required for the deallylation.

19. Process according to Claims 11 to 18, **characterized in that** the amount of the said nucleophile is at least equal to a value between 0.9 and 1.1 times the amount stoichiometrically required for the deallylation.

20. Process according to Claims 11 to 19, **characterized in that** the said nucleophile contains an acid function.

21. Process according to Claims 11 to 20, **characterized in that** the said nucleophile contains a nucleophilic function which itself contains a sulphur atom as nucleophilic atom.